Europäisches Patentamt

European Patent Office

Office européen des brevets

(11) Publication number: **0 519 437 A1**

# EUROPEAN PATENT APPLICATION

(21) Application number: 92110251.3

(22) Date of filing: 17.06.92

(51) Int. Cl.5: **A01N 63/00**, C12N 1/20,
//(C12N1/20,C12R1:39),
(C12N1/20,C12R1:40)

(30) Priority: 20.06.91 IT MI911699

(43) Date of publication of application:
23.12.92 Bulletin 92/52

(84) Designated Contracting States:
AT BE CH DE DK ES FR GB GR IT LI LU MC
NL PT SE

(71) Applicant: **MINISTERO DELL' UNIVERSITA' E
DELLA RICERCA SCIENTIFICA E
TECNOLOGICA**
76, Lungotevere Thaon di Revel
I-00196 Roma(IT)

(72) Inventor: **Tolentino, Daniela**
**Via Marciano, 10**
**I-20133 Milano(IT)**
Inventor: **Cassinelli, Clara**
**Via S. Martino, 23**
**I-14040 Cortiglione (Asti)(IT)**
Inventor: **Orlandini, Luisa**
**Via Roma 84**
**I-28060 S. Nazzaro Sesia (Novara)(IT)**
Inventor: **Noris, Emanuela**
**Corso Ferraris 137**
**I-10128 Torino(IT)**
Inventor: **Signorini, Ernesto**
**Via Matteotti, 51**
**I-21046 Malnate (Varese)(IT)**
Inventor: **Pirali, Giorgio**
**Via Ticino, 2**
**I-21047 Saronno (Varese)(IT)**

(74) Representative: **von Hellfeld, Axel, Dr.
Dipl.-Phys. et al
Wuesthoff & Wuesthoff Patent- und
Rechtsanwälte Schweigerstrasse 2
W-8000 München 90(DE)**

(54) Bacterial pseudomonas strains (NCIMB 40403, NCIMB 40376, NCIMB 40375, NCIMB 40377, NCIMB 40402, NCIMB 40379) capable of controlling fungal infections.

(57) Microorganisms belonging to Pseudomonas genus (NCIMB 40403, NCIMB 40376, NCIMB 40375, NCIMB 40377, NCIMB 40402, NCIMB 40379) are disclosed, together with their use for controlling phytopathogenic fungi, in particular Pythium spp. and/or Fusarium spp., which strike a wide range of crops.

The present invention relates to microorganisms belonging to Pseudomonas genus (NCIMB 40403, NCIMB 40376, NCIMB 40375, NCIMB 40377, NCIMB 40402, NCIMB 40379) and to their use in agricultural field in order to control phytopathogenic fungi, in particular Pythium spp. and/or Fusarium spp., which are well-known phytopathogenic agents, with consequent improvement in crop vegetative growth and production.

Several agents of fungal nature are the cause of a large number of soil diseases, which cause very considerable damages to most crops.

Some of these phytopathogenic agents cause the most serious damages during the first stages of plant growth, with a resulting decrease in seed germination, or attacking the seedling already at hypocotyl level, causing sore shin and root system moulds; other of them, on the contrary, mainly act during the subsequent stages of plant growth, causing the struck plants to underdevelop, and resulting into an end loss in production.

In particular, the species belonging to genera Fusarium and Pythium are considerably important from phytopathological viewpoint, owing to the large number of host vegetable species struck by them and due to their wide geographic distribution, prevailingly in regions with temperate, warm, high-humidity climates.

Among the alterations induced by Fusarium, the most considerable ones are those which concern the conducting vessels (e.g., tracheofusariosis); equally important are the mould processes involving fleshy organs (fruits, tubers, bulbs and so forth), or herbaceous organs, and the cancers affecting wooden organs.

Moreover, these alterations can sometimes be associated with each other and variously combined.

The development of the diseases caused by the species belonging to Fusarium and Pythium genera is often favoured by bad pedological conditions (acidic, impermeable, moist soils) and is also favoured by the simultaneous presence of phytophagous organisms which, causing lesions to the host, favour an easier establishment thereof.

The wide range of hosts, a very efficient system of persistence in saprophytic stage and the frequent settlement in hystological seats difficult to be reached by most common pesticides, cooperate to render the fight against these microorganisms, a particularly difficult one.

In general, the more commonly suggested strategies against such pathogenic agents are the indirect ones, such as, e.g., the practice of crop rotation, the use of resistant cultivars, the correction of acidic soil pH by means of the addition of limestone and the resort to all those agricultural strategies which are per se favourable to the concerned crops.

At present, also the disinfection of soil with vapour or fume generating chemicals, is used.

The effectiveness of these treatments is reduced by their poor specificity and their too high costs for open-field crops.

Other actions carried out with chemical products, such as Galben(R) (Agrimont), Apron(R) and Ridomil(R) - (Ciba Geigy) and Previcur(R) (Schering) against such pathogenic agents not always reach the desired effectiveness: instability on soil, lack of systemic transfer from roots and arising of resistance, are among the most common drawbacks.

The use of microorganisms as the means for controlling pests has been known for long. More recently, many investigations have been carried out by studying soils which are natural suppressants for such diseases.

Several effective bacteria were isolated, among which there are also species belonging to Pseudomonas genus, which display a control activity for infections caused by phytopathogenic agents on a wide range of crops.

The primary seat of growth and diffusion of the above said bacteria is soil in which, together with such other microorganisms as fungi and bacteria, they generally perform functions of metabolization of the organic matter present in soil.

Particular strains of Pseudomonas are known, such as, e.g., those as disclosed by R.M. Osburn et al., Ecology and Epidemiology, Vol. 79, No. 6, 1989, pages 709-716 and C.R. Howell and R.D. Stipanovic, Phytopathology, Vol. 70, No. 8, 1980, pages 712-715, which, when applied to seeds of sugar beets or cotton, or to the soil of the above said crops, showed a certain capability of controlling infections caused by Pythium ultimum.

One among the most common problems to be solved in this regard, is developing a system of administration of the beneficial agent, which is capable of enhancing the action effectiveness and the survival capability thereof.

The results obtained with such microorganisms evidence a specificity of the system consisting of plant, phytopathogenic agent and biological control agent, which limits the use of the latter to particular conditions.

Furthermore, owing to the peculiarity of the living being which is the disease control agent, such a type of intervention showed often to be less effective than chemicals.

Therefore, the need was felt for finding out, in microbiological field, bacterial strains which are capable of controlling infections caused by phytopathogenic agents, which appear on different plants and have a comparable effectiveness to the above cited chemicals.

We have found now Pseudomonas strains, which, as such or as biologically pure cultivations, constitute a first object of the present invention, identified as NCIMB 40403, NCIMB 40376, NCIMB 40375, NCIMB 40377, NCIMB 40402, NCIMB 40379 and having special morphological, biochemical and physiological characteristics, which, when are added to the soil on which the crops are cultivated, or are used as dressings for seeds or are anyway brought into contact with the roots of the plants, control the infections caused by fungi, without showing the limits which affect the microorganisms known from the prior art, with the germination rate of seeds, the vegetation growth and crop production being consequently improved.

A further object of the instant invention is a method for treating crops, infested in particular by Pythium spp. and/or Fusarium spp., which method consists of treating the seeds, the roots of said crops, or the soil on which said crops grow, with strains of Pseudomonas NCIMB 40403, NCIMB 40376, NCIMB 40375, NCIMB 40377, NCIMB 40402 and NCIMB 40379, in the form of cells, as such, or as suitable formulations.

The bacteria of Pseudomonas genus, which are the subject-matter of the present invention, have shown a fungicidal action which was tested, in vitro, on a wide range of phytopathogenic agents, such as: Pythium ultimum, Rhizoctonia solani, Sclerotium rolfsii, Fusarium oxysporum lycopersici, Fusarium roseum, Fusarium culmorum, Fusarium graminearum, Fusarium avenaceum, Microdochium nivale (formerly "Fusarium nivale"), Verticillium dahliae, Thielaviopsis basicola, Alternaria tenuis, Phoma glomerulata, Rosellinia necatrix.

At a later time, during tests, first carried out in greenhouse and then on open-field crops, the above said bacteria showed to be active mainly against fungi belonging to Pythium and/or Fusarium species, and can be applied to a wide range of crops, such as leguminous plants (bean, pea, soy), cucumbers, cotton, sugar beets, wheat (Triticum durum), tomatoes and corn.

Furthermore, their action is particularly beneficial because it takes place with an analogous, and sometimes higher, effectiveness as compared to most commonly used chemicals.

The morphological, biochemical and physiological characteristics determined on Pseudomonas strains NCIMB 40403, NCIMB 40376, NCIMB 40375, NCIMB 40377, NCIMB 40402 and NCIMB 40379 made it possible said strains to be differentiated from Pseudomonas strains known heretofore.

For such determinations, said Pseudomonas strains NCIMB 40403, NCIMB 40376, NCIMB 40375, NCIMB 40377, NCIMB 40402 and NCIMB 40379 were isolated from rhyzosphere of crops grown on repressive soils, i.e., soils on which symptoms of phytopathies from telluric origin were not evidenced.

By "repressive soil", a soil is meant in which, after a time period during which disease symptoms were manifested, an infection cannot be demonstrated during several subsequent years.

Strain NCIMB 40375, listed for internal laboratory use under the conventional code PD 30, was isolated from a sample drawn from a corn field sited within the territory of the province of Verona; strain NCIMB 40376, listed for internal laboratory use under the conventional code PD 57, was isolated from a sample drawn from a tomato field sited within the territory of the province of Verona; strain NCIMB 40403, listed for internal laboratory use under the conventional code PD 288, was isolated from a sample drawn from a grapevine species sited at Niclara Cortara locality within the territory of the province of Bolzano; strain NCIMB 40379, listed for internal laboratory use under the conventional code PD 259, was isolated from a sample drawn from the root bast of an apple-tree, within the territory of the province of Bolzano; strain NCIMB 40402, listed for internal laboratory use under the conventional code PD 173, was isolated from a sample drawn from mais root within the territory of the province of Novara; strain NCIMB 40377, listed for internal laboratory use under the conventional code PD 108, was isolated from a sample drawn from potato rhyzosphere within the territory of the province of Trento.

The sample, consisting of portions of roots and of surrounding soil, was suspended in 0.01 M phosphate buffer, and serial dilutions were distributed on agarized growth substrates known to those skilled in the art to be selective for Pseudomonas genus such as, e.g., NPC substrate (wherein by "NPC", the King's B Agar culture substrate is meant, to which the following antibiotics are added in order to increase the selectivity thereof: 45 mg of novobiocin per litre, 75 x $10^3$ units of penicillin G per litre and 100 mg of cycloheximide per litre) and containing, per each litre of $H_2O$, 20 g of proteose peptone, 10 ml of glycerol, 1.5 g of $MgSO_4.7H_2O$, 1.5 g of $K_2HPO_4$.

The cultures of said microorganisms were filed on April 11th, 1991, under the Covenant of Budapest, with the National Collection of Industrial Bacteria (c/o The National Collection of Industrial and Marine Bacteria Ltd., Torry Research Station, P.O. Box 31, 135 Abbey Road, Aberdeen AB 98 DG, Scotland, U.K.), where the following access codes were respectively assigned to them: NCIMB 40375, NCIMB 40376, NCIMB 40403, NCIMB 40377, NCIMB 40402 and NCIMB 40379.

After being grown at 28°C, the Pseudomonas strains identified in that way can be maintained on common substrates such as, e.g., Nutrient Agar or King's B Agar (KB) at 25-30°C and can be propagated by means of techniques per se known to those skilled in the art; on such substrates, they furthermore produce a fluorescent pigment visible by exposure to UV light at 365 nm.

The above said colonies of Pseudomonas strains are of circular shape with convex edge, smooth surface and grainy consistency.

The cells have the shape of a non-sporogenic, Gram-negative, very mobile rod, of 1-2 μm of diameter and 3-4 μm of length.

The Pseudomonas strains according to the present invention can grow within a wide temperature range, i.e., from 4 to 40°C; however, the optimal growth temperature is comprised within the range of from 25 to 30°C.

On the basis of their morphological, physiological, biochemical characteristics, and of their characteristics of susceptibility to antibiotics, as reported in the following tables, the Pseudomonas strains in vegetation stage were identified as: PD 30 (NCIMB 40375), i.e., Pseudomonas putrida, PD 57 (NCIMB 40376), and PD 288 (NCIMB 40403), i.e., Pseudomonas fluorescens, PD 108 (NCIMB 40377), PD 173 (NCIMB 40402) and PD 259 (NCIMB 40379), i.e., Pseudomonas maltophilia, in accordance with Bergey's Manual of Systematic Bacteriology, 1989.

Table 1a

| Morphology of the colonies of strains PD 30, PD 57, PD 288 | | | |
|---|---|---|---|
| Characteristics | PD 30 | PD 57 | PD 288 |
| Size (mm) | 2-4 | 2-4 | 2-4 |
| Shape | Circular | Circular | Circular |
| Edge | Full | Full | Full |
| Relief | Convex | Pulvinate | Convex |
| Surface | Glossy | Glossy | Matt |
| Transparency | Not transparent | Not transparent | Not transparent |
| Consistency | Creamy | Creamy | Creamy |
| Colour | Yellow | Yellow | Ivory |
| Odour | Strong | Strong | Strong |

Table 1b

| Morphology of the colonies of strains PD 108, PD 173, PD 259 | | | |
|---|---|---|---|
| Characteristics | PD 108 | PD 173 | PD 259 |
| Size (mm) | 0.5-2 | 0.5-2 | 0.5-2 |
| Shape | Punctiform | Punctiform | Punctiform |
| Edge | Full | Full | Full |
| Relief | Flat | Flat | Flat |
| Surface | Matt | Matt | Matt |
| Transparency | Not transparent | Not transparent | Not transparent |
| Consistency | Creamy | Creamy | Creamy |
| Colour | Cream | Cream | Cream |
| Odour | Strong | Strong | Strong |

Table 2a

| Cellular morphology of strains PD 30, PD 57, PD 288 | | | |
|---|---|---|---|
| Characteristics | PD 30 | PD 57 | PD 288 |
| Length ($\mu$M)* | 1.5-4 | 1.5-4 | 1.5-4 |
| Length/Diameter ($\mu$M) | 0.5-1 | 0.5-1 | 0.5-1 |
| Shape | Rods | Rods | Rods |
| Mobility | Present | Present | Present |
| Arrangement | Single cells | Single cells | Single cells |
| Formation of spores | Absent | Absent | Absent |

Table 2b

| Cellular morphology of strains PD 108, PD 173, PD 259 | | | |
|---|---|---|---|
| Characteristics | PD 108 | PD 173 | PD 259 |
| Length ($\mu$M)* | 0.5-1.5 | 0.5-1.5 | 0.5-1.5 |
| Length/Diameter ($\mu$M) | 0.5-1 | 0.5-1 | 0.5-1 |
| Shape | Rods | Rods | Rods |
| Mobility | Present | Present | Present |
| Arrangement | Single cells | Single cells | Single cells |
| Formation of spores | Absent | Absent | Absent |

## Table 3a

## Physiological and biochemical tests on strains

## PD 30, PD 57, PD 288

| Characteristics | PD 30 | PD 57 | PD 288 |
|---|---|---|---|
| Gram staining | − | − | − |
| Catalase | + | + | + |
| Oxidase | + | + | + |
| Arginine dehydrolase | + | + | + |
| Gelatinase | − | + | + |
| Pectolytic capability (CVP substrate) | − | − | +/− |
| Urease (Christensen substrate) | + | + | + |
| Methyl red and Voges-Proskauer | − | − | − |
| Formations of levanes | − | − | − |
| Growth on NaCl at 6.5% | − | − | − |
| Growth on Cetrimide at 1% | − | − | − |
| Production of H₂S (TSI substrate) | − | − | − |
| Indole production | − | − | − |
| Citrate use (Christensen and Simmons substrate) | + | + | + |
| Production of pigments: | | | |
| − pyoverdine | + | + | + |
| − pyocyanine | − | − | − |

| | | | |
|---|---|---|---|
| Lysine decarboxylase | − | − | − |
| Starch hydrolysis | − | − | − |
| Lipase | + | + | + |
| Lecithinase | + | − | + |
| Esculine hydrolysis | − | − | − |
| Oxidation/Fermentation: * | | | |
| − glucose | + | + | + |
| − saccharose | − | + | − |
| − maltose | − | − | − |
| − mannitol | + | + | + |
| − lactose | − | − | − |
| − inositol | − | − | − |
| − mannose | + | + | + |
| − fructose | + | + | + |
| − arabinose | + | + | + |
| − xylose | + | + | + |
| Growth on MacConkey substrate | + | + | + |
| Growth at 4°C | + | + | + |
| Growth at 41°C | − | − | − |

* None of strains shows the sugar fermentation metabolism

## Table 3b

## Physiological and biochemical tests on strains

## PD 108, PD 173, PD 259

| | PD | PD | PD |
|---|---|---|---|
| **Characteristics** | **108** | **173** | **259** |
| **Gram staining** | − | − | − |

| | | | |
|---|---|---|---|
| Catalase | + | + | + |
| Oxidase | − | − | − |
| Arginine dehydrolase | − | − | − |
| Gelatinase | + | + | + |
| Pectolytic capability (CVP substrate) | − | + | − |
| Urease (Christensen substrate) | − | − | − |
| Methyl red and Voges-Proskauer | − | − | − |
| Formations of levanes | − | − | − |
| Growth on NaCl at 6.5% | − | − | − |
| Growth on Cetrimide at 1% | − | − | − |
| Production of $H_2S$ (TSI substrate) | − | − | − |
| Indole production | − | − | − |
| Tryptophan deaminase | − | − | − |
| Citrate use (Christensen and Simmons substrate) | − | − | − |
| Production of pigments: | | | |
| − pyoverdine | − | − | − |
| − pyocyanine | − | − | − |
| Lysine decarboxylase | + | + | + |
| Ornithine decarboxylase | − | − | − |
| Starch hydrolysis | − | − | − |
| Lipase | + | + | + |
| Lecithinase | − | − | − |
| Esculine hydrolysis | + | + | + |
| β-galactosidase | − | + | + |

| | | | |
|---|---|---|---|
| Reduction of NO₃ into NO₂ | − | − | − |
| Reduction of NO₃ into N₂ | − | − | − |
| Use of: | | | |
| − glucose | + | + | + |
| − arabinose | − | − | − |
| − mannose | + | + | + |
| − mannitol | − | − | − |
| − N-acetyl-glucosamine | + | + | + |
| − maltose | + | + | + |
| − gluconate | − | − | − |
| − caproate | − | − | − |
| − adipate | − | − | − |
| − malate | + | + | + |
| − citrate | + | + | + |
| − phenylacetate | − | − | − |
| Oxidation/Fermentation: * | | | |
| − glucose | + | + | + |
| − saccharose | + | +/− | +/− |
| − maltose | + | + | + |
| − mannitol | − | − | − |
| − lactose | +/− | +/− | + |
| − inositol | − | − | − |
| − mannose | + | + | + |
| − fructose | + | + | + |
| − arabinose | − | − | − |

9

– xylose                                           –     –     –

Growth on MacConkey substrate        +    +    +

Growth at 4°C                             +    +    +

Growth at 41°C                         –     –     –

* None of strains shows the sugar fermentation metabolism

Table 4a

| Antibiogram of strains PD 30, PD 57, PD 288 | | | | |
|---|---|---|---|---|
| Antibiotic (BBL) | Dose (μg) | PD 30 | PD 57 | PD 288 |
| Nalidixic acid | 30 | R (2) * | I (16) | R |
| Ampicillin | 10 | R | R | R |
| Bacitracin | 10 | R | R | R |
| Cephaloridine | 30 | R | R | R |
| Chloramphenicol | 30 | R | R | R |
| Chlorotetracycline | 30 | R (3) | R (11) | R |
| Erythromycin | 15 | R | R | R |
| Fosfomycin | 50 | S (15) | S (17) | S (17) |
| Gentamycin | 10 | R (4) | S (16) | S (17) |
| Kanamycin | 30 | I (13) | S (20) | S (20) |
| Lincomycin | 2 | R | R | R |
| Neomycin | 30 | R (4) | S (19) | S (18) |
| Novobiocin | 30 | R | R | R |
| Oleandomycin | 15 | R | R | R |
| Oxytetracycline | 30 | R | R (14) | R |
| Penicillin | 10 | R | R | R |
| Rifamycin | 30 | R | R (10) | R (10) |
| Rifampicin | 30 | R (6) | S (14) | S (14) |
| Streptomycin | 10 | R | R | R |
| Tetracycline | 30 | R | R (10) | R (14) |
| Tobramycin | 10 | R (5) | S (17) | S (18) |
| Vancomycin | 30 | R | R | R |

## Table 4a

## Antibiogram of strains PD 108 PD 173, PD 259

| Antibiotic (BBL) | Dose (µg) | PD 108 | PD 173 | PD 259 |
|---|---|---|---|---|
| Nalidixic acid | 30 | S (17) * | R | S (17) |
| Ampicillin | 10 | R | R | R |
| Bacitracin | 10 | R | R | R |
| Cephaloridine | 30 | R | R | R |
| Chloramphenicol | 30 | R (10) | R | R (8) |
| Chlorotetracycline | 30 | R | R | R (6) |
| Erythromycin | 15 | R | R | R |
| Fosfomycin | 50 | R | R | R |
| Gentamycin | 10 | S (17) | R | R (3) |
| Kanamycin | 30 | S (17) | R | R (4) |
| Lincomycin | 2 | R | R | R |
| Neomycin | 30 | S (17) | R | R |
| Novobiocin | 30 | R | R | R |
| Oleandomycin | 15 | R | R | R |
| Oxytetracycline | 30 | R | R | R (3) |

```
Penicillin          10      R           R           R
Rifamycin           30      R (12)      R (2)       I (15)
Rifampicin          30      S (20)      R (2)       I (15)
Streptomycin        10      I (15)      R           R
Tetracycline        30      R           R           R (6)
Tobramycin          10      I (15)      R           R (4)
Polymyxin B         300     R (10)      R           R (7)
Vancomycin          30      R           R           R
```

* Values in brackets are the inhibition halos, as mm.

– Evaluation of the level of sensibility to antibiotics:

R = resistant;

I = intermediate;

S = sensible.

[According to NCCLS (National Committee for Clinical Laboratory Standards].

The strains taken into consideration display furthermore particular metabolic characteristics, involved in the activity of control of phytopathogenic fungi, as disclosed in Tables 5a and 5b.

Table 5a

| Metabolic characteristics of strains PD 30, PD 57, PD 288 | | | |
|---|---|---|---|
| Characteristics | PD 30 | PD 57 | PD 288 |
| Production of gas substances with antibiotic activity | - | - | + |
| Production of siderophores | + | + | + |
| Production of antibiotics | - | - | + |

Table 5b

| Metabolic characteristics of strains PD 108, PD 173, PD 259 | | | |
|---|---|---|---|
| Characteristics | PD 108 | PD 173 | PD 259 |
| Production of gas substances with antibiotic activity | + | + | + |
| Production of siderophores | - | - | - |
| Production of antibiotics | + | + | + |

Cultural characteristics, and treatment modalities

Pseudomonas NCIMB 40403, NCIMB 40376, NCIMB 40375, NCIMB 40377, NCIMB 40402 and NCIMB 40379 can be cultivated in shaken culture on usual liquid substrates, or in stationary culture on agarized substrate slab, at a temperature preferably comprised within the range of from 25°C to 30°C.

Directly from the liquid culture, or by re-suspending the microorganisms from the slab culture, a bacterial suspension with $10^5$-$10^9$ CFU/ml is formed (CFU = colony forming units).

The treatment is carried out by seed dressing, using the bacterial suspension as such, or after suitable formulation.

The formulation can contain from $10^3$ to $10^{10}$ bacterial cells per gram of liquid or solid formulation.

Non-aqueous formulations comprise the use of inert supports acceptable for agricultural use, such as, e.g., vermiculite, sepiolite, celite or kaolin, coadjuvated by adhesion promoters, such as gum arabic, carboxymethylcellulose (CMC) or poly(vinyl alcohol) (PVA).

The use of poly(vinyl alcohol), in particular PVA N300, resulted to be the most interesting one, because the other adhesion promoters, besides securing a good survival of beneficial microorganisms, also allow an excessive development of most phytopathogenous fungi to be controlled by means of the treatment.

The seed were first impregnated with poly(vinyl alcohol) and then were coated with the formulated composition obtained by mixing an aliquot of liquid suspension of Pseudomonas with finely ground vermiculite.

The seeds were let dry overnight at room temperature and were placed on agarized substrate King's B Agar (KB) substrate in order to monitor the development of microorganisms contained in seed envelope.

From this test, it was seen that the above said formulation technique is valuable as in order to produce seeds coated with Pseudomonas for greenhouse tests, as well as for open-field tests.

The formulation supported on vermiculite can be directly applied to seeds, to the surrounding soil or to the roots system at transplantation time.

The bacterial strains of Pseudomonas NCIMB 40403, NCIMB 40376, NCIMB 40375, NCIMB 40377, NCIMB 40402 and NCIMB 40379 can be used either individually, or mixed with one another.

Some examples are reported now in the following, for the purpose of illustrating the present invention, without limiting it.

Example 1

Phytopathogenic agents-antagonist activity in vitro

This example demonstrates the wide range of activity of inhibition of phytopathogenic fungi displayed by Pseudomonas strains NCIMB 40403, NCIMB 40376, NCIMB 40375, NCIMB 40377, NCIMB 40402 and NCIMB 40379.

The activity of inhibition of growth of phytopathogenic fungi in vitro was determined by inoculating the bacterial strains of Pseudomonas NCIMB 40403, NCIMB 40376, NCIMB 40375, NCIMB 40377, NCIMB 40402 and NCIMB 40379 along the outermost perimeter of a slab containing agarized King's B Agar (KB) substrate, then keeping the slabs at 28°C in order to allow the bacterial growth to take place.

Twenty-four hours later, a portion of phytopathogenic fungus is inserted in the centre of the slab, and the slab is stored at 25°C in order to favour the growth of the fungus.

After 3 days, the agonist activity is measured in terms of inhibition of the diameter of mycelial growth of phytopathogenic fungus, as compared to a bacterium-free control.

By means of such a test, the Pseudomonas strains NCIMB 40403, NCIMB 40376, NCIMB 40375, NCIMB 40377, NCIMB 40402 and NCIMB 40379 caused a decrease of ≥50% in mycelial growth on a wide range of phytopathogenic fungi (see Table 6).

Table 6

| Type of fungus | PD30 | PD57 | PD288 | PD108 | PD173 | PD259 |
|---|---|---|---|---|---|---|
| P. ultimum | + | + | + | + | + | + |
| F. roseum | - | - | + | - | + | + |
| F. ox. lyc. | - | - | + | + | + | + |
| F. culmorum | - | - | - | + | + | + |
| F. graminearum | - | - | - | + | + | + |
| F. avenaceum | - | - | + | + | + | + |
| M. nivale (formerly F. nivale) | + | + | + | + | + | + |
| R. solani | - | + | + | + | - | + |
| S. rolfsii | + | - | + | + | + | + |
| P. glomerulata | + | + | + | + | + | + |
| V. dahliae | - | - | + | - | - | + |
| T. basicola | - | - | + | - | - | + |
| A. tenuis | - | - | + | - | - | - |
| R. necatrix | + | - | + | + | + | + |
| B. cinerea | + | + | + | + | + | + |

Example 2

Protection from infection caused by P. ultimum and other phytopathogenic agents, by means of Pseudomonas strains NCIMB 40403, NCIMB 40376 and NCIMB 40375

BEAN

This example demonstrates the capability of Pseudomonas strains NCIMB 40403, NCIMB 40376 and NCIMB 40375, of controlling the infection caused by pathogenic fungi on bean plants under greenhouse conditions.

Bean seeds ("Lingua di fuoco" cuitivar) are treated by immersion into a bacterial suspension of $10^6$-$10^9$ CFU/ml (CFU = colony forming units) containing carboxymethylcellulose (CMC) at 1% and 1 M $MgSO_4$ mixed in the ratio of 1:1.

After being inoculated in that way, the seeds are let soak for at least 1 hour at room temperature, before sowing.

Sowing takes place in pots of 10 cm of diameter, each containing 500 g of soil.

The soil can be either naturally or artificially infected with P. ultimum or R. solani or T. basicola; the level of fungal infection should anyway correspond to a content of at least 300 fungal propagules per gram of soil.

Each pot contains 5 seeds buried under the soil surface, at a depth of 1-2 cm.

After sowing, the pots are stored inside chambers thermostatted at 23°C with 60% R.H. (relative humidity) and photoperiod of 16 lighting hours.

The controls are constituted by untreated seeds, and seeds treated with a solution of an antifungal agent (Apron[R] or Previcur[R] or Ridomil[R]).

Per each thesis, at least five replicate tests were carried out.

The degree of germination and of resistance to infection were evaluated 15 days later and were measured by using a resistance index:

0 = not germinated seed;
1 = seed germinated but plant dead;
2 = plant affected by pest;
3 = plant affected by pest but with slight brown spots;
4 = healthy plant.

The index values reported as results (minimal value 0, maximal value 20) are the average resistance index of each thesis.

The seeds treated with Pseudomonas strains NCIMB 40403, NCIMB 40376 and NCIMB 40375 show a considerably higher resistance index than of untreated seeds, of seeds treated with the chemical, and of seeds treated with strain R20 (R.M. Osburn et al., 1989) and with DG[R] (Dagger G[R] ECOGEN) (see Table 7).

Table 7

| Infection resistance index of bean seeds | | | | | | | |
|---|---|---|---|---|---|---|---|
| Control | Carbovax | Apron/Previc. | PD30 | PD57 | PD288 | R20 | DG |
| *) 5.2 | -- | 13.6/3.0 | 9.2 | -- | 17 | 6.2 | 12.2 |
| **) 0.8 | 3.2 | -- | -- | 1.6 | 3.8 | 0.8 | 0.6 |

*) Infection caused by P. ultimum
**) Infection caused by R. solani

PEA

This example demonstrates the capability of Pseudomonas strains NCIMB 40403, NCIMB 40376 and NCIMB 40375, of controlling the infection caused by P. ultimum on pea plants.

The test was carried out analogously to the above example carried out on bean, using pea seeds ("Nano Meraviglia d'Italia" cultivar).

Also in this case, the strains of Pseudomonas NCIMB 40403, NCIMB 40376 and NCIMB 40375 considerably increased the resistance of pea seeds to the infection caused by P. ultimum, analogously to the synthetic fungicide used (Previcur[R] ) (see Table 8).

Table 8

| Index of resistance to infection caused by P. ultimum, shown by pea seeds after treatment with PD 288 | | | | | | |
|---|---|---|---|---|---|---|
| | Control | Previcur[R] | PD288 | PD 57 | R20 | DG[R] |
| Greenhouse | 2.8 | 11.0 | 11.8 | -- | 1.6 | 2.8 |
| Open field | 38,2 | 66.4 | 56.2 | 54.4 | -- | 48.6 |

The open-field tests were carried out under infection conditions analogous to those adopted for greenhouse tests, and using field sections with 40 plants each (minimal value of Resistance Index = 0, maximal value = 160).

SOY

This example demonstrates the capability of Pseudomonas strains NCIMB 40403, NCIMB 40376 and NCIMB 40375, of controlling the infection caused by pathogenic fungi on soy plants.

The test was carried out analogously to the above examples using soy seeds ("Sakai" cultivar) (see Table 9).

Table 9

| Infection resistance index of soy seeds after treatment with PD 288 | | | | | | |
|---|---|---|---|---|---|---|
| | Control | Captan | Previcur | PD288 | R20 | DG |
| P. ultimum | 3.6 | -- | 14.2 | 11.6 | 3.8 | 5.2 |
| T. basicola | 9.6 | 16.6 | -- | 12.4 | 11.6 | 7.8 |

CUCUMBER

This example demonstrates the capability of Pseudomonas strains NCIMB 40403, NCIMB 40376 and NCIMB 40375, of controlling the infection caused by P. ultimum on cucumber plants, still under greenhouse

EP 0 519 437 A1

conditions.

The test was carried out analogously to the above examples using cucumber seeds ("Marketer Long" cultivar) (see Table 10).

<u>Table 10</u>

<u>Index of resistance of cucumber seeds to the infection by</u>

<u>P. ultimum after treatment with PD 288, PD 30, PD 57</u>

Apron[R]     1

Previcur[R] 2

| Control | Ridomil[R]  3 | PD 30 | PD 57 | PD288 | R20 | DG |
|---|---|---|---|---|---|---|
| 12.4 | 1 = 21.6 | 17 | 18,0 | 23,8 | 15,2 | 14 |
|  | 2 = 22 |  |  |  |  |  |
|  | 3 = 17 |  |  |  |  |  |

The seeds treated with Pseudomonas strains NCIMB 40403, NCIMB 40376 and NCIMB 40375 display a considerably higher value of Resistance Index than untreated seeds, or seeds treated with the chemical agent.

SUGAR BEET

This example illustrates the capability of Pseudomonas strains NCIMB 40403, NCIMB 40376 and NCIMB 40375, of controlling the infection caused by P. ultimum on sugar beet.

Single-germ sugar beet seeds ("Extramonosaurus" cultivar) were treated analogously to the preceding examples.

The measure of control of infection by P. ultimum is expressed as percentage of reduction in disease (see Table 11).

Table 11

| Control | Previcur[R] | PD 57 | PD 288 |
|---|---|---|---|
| 0 | 92 | 66 | 71 |

COTTON

This example illustrates the capability of Pseudomonas strains NCIMB 40403, NCIMB 40376 and NCIMB 40375, of controlling the infection caused by P. ultimum on cotton.

Cotton seeds ("Sindos 80" cultivar) were treated analogously to the preceding examples (see Table 12).

16

Table 12

| Index of resistance of cotton seeds to the infection caused by P. ultimum after treatment with PD 288 | | | | | | | |
|---|---|---|---|---|---|---|---|
| Control | Apron[R]/Previcur[R] | Carbov. | PD30 | PD57 | PD288 | R20 | DG |
| *) 1.0 | 4.4 / 6.0 | -- | 3.6 | 3.0 | 4.6 | 0.4 | 1.8 |
| **) 3.4 | -- | 6.2 | -- | 4.0 | 5.4 | 3.8 | 8.2 |

\*) Infection caused by P. ultimum
\*\*) Infection caused by R. solani

The seeds treated with Pseudomonas strains NCIMB 40403, NCIMB 40376 and NCIMB 40375 display a better value of Resistance Index than control seeds, and, in the case of seeds treated with PD 288 strain, higher than as obtained with the chemical treatment.

Example 3

Protection from infection caused by Fusaria spp., by means of Pseudomonas strains NCIMB 40377, NCIMB 40402 and NCIMB 40379

WHEAT (TRITICUM DURUM)

This example demonstrates the capability of Pseudomonas strains NCIMB 40377, NCIMB 40402 and NCIMB 40379, of controlling the infection caused by Microdochium nivale (formerly Fusarium nivale) by means of the "seedling symptom test" on wheat (Triticum durum).
Laboratory tests were carried out on agarized substrate incubated at 15°C (see Table 13).

Table 13

| | Treatment | Germinated seeds | | Non germinated seeds |
|---|---|---|---|---|
| | | Healthy Seedlings | Infected Seedlings | |
| 1st Observation (8 days) | Control | 58 % | 19 % | 23 % |
| | PD 108 | 78 % | 5 % | 17 % |
| | PD 259 | 67 % | 4 % | 29 % |
| 2nd Observation (16 days) | Control | 39 % | 38 % | 2 % |
| | PD 108 | 53 % | 30 % | 17 % |
| | PD 259 | 50 % | 26 % | 24 % |
| 3rd Observation (22 days) | Control | 16 % | 61 % | 23 % |
| | PD 108 | 27 % | 56 % | 17 % |
| | PD 259 | 35 % | 42 % | 23 % |

TOMATO

This example demonstrates the capability of Pseudomonas strains NCIMB 40377, NCIMB 40402 and NCIMB 40379, of controlling the infection caused by Fusarium oxysporum lycopersici on tomato, under greenhouse conditions.
For the test, an infection by Fusarium oxysporum lycopersici was caused at transplantation time on tomato ("Cuore di bue" cultivar).
One month later, the development of the aerial portion and of the root system of tomato plants was evaluated, by determining the dry weight of each thesis (see Tables 14a and 14b).

## Table 14a

### Transplantation onto vermiculite

| | Weight (g) | |
| --- | --- | --- |
| Treatment | Roots | Stem + leaves |
| Control | 1.182 | 1.198 |
| R 20 | 3.092 * | 1.174 * |
| PD 108 | 4.62 ** | 1.154 |
| PD 173 | 3.334 ** | 1.412 |
| PD 259 | 3.684 ** | 1.724 * |
| PD 288 | 5.466 ** | 1.970 ** |

## Table 14b

### Transplantation into soil

| Treatment | Total weight of stem + leaves (g) |
| --- | --- |
| Control | 0.09 |
| R20 | 0.73 * |
| PD 108 | 0.6 * |
| PD 173 | 0.57 * |
| PD 259 | 0.39 |
| PD 288 | 0.23 |
| Carbendazim | 0.27 |

N.B. - The data obtained were submitted to statistic processing (variance analysis, LSD test). From the comparison with the respective controls, some values result to be significant (*) and highly significant (**) from the statistic viewpoint.

CORN

This example demonstrates the capability of Pseudomonas strains NCIMB 40377, NCIMB 40402 and NCIMB 40379, of controlling the infection caused by spontaneous Fusaria spp. on corn seed ("Plenus" cultivar).

Laboratory tests were carried out on agarized substrate, incubated at 23°C (see Table 15).

Table 15

| Treatment | Decrease in infection, % | |
|-----------|------------|------------|
| | Test 1 | Test 2 |
| R20 | 20 % | 42 % |
| PD 259 | 14 % | 37 % |
| PD 30 | 44 % | 30 % |
| Captan | 44 % | 64 % |

**Claims**

1. Pseudomonas strains identified with the collection codes NCIMB 40403, NCIMB 40376, NCIMB 40375, NCIMB 40377, NCIMB 40402 and NCIMB 40379 and filed on April 11th, 1991 with The National Collection of Industrial and Marine Bacteria of Aberdeen, or biologically pure cultures thereof.

2. Pseudomonas strains according to claim 1, characterized by a growth temperature comprised within the range of from 4 to 40°C, preferably comprised within the range of from 25 to 30°C.

3. Formulation for controlling the infections caused by phytopathogenic fungi, comprising at least one Pseudomonas strain selected from among those identified with the codes NCIMB 40403, NCIMB 40376, NCIMB 40375, NCIMB 40377, NCIMB 40402 and NCIMB 40379.

4. Formulation for controlling the infections caused by phytopathogenic fungi, according to claim 3, characterized in that, when such phytopathogenic fungi are Pythium spp. and/or Fusarium spp., said formulation comprises Pseudomonas strains NCIMB 40403, NCIMB 40376, NCIMB 40375, NCIMB 40377, NCIMB 40402 and NCIMB 40379 in a concentration comprised within the range of from $10^3$ to $10^{10}$ bacteria per gram of formulation.

5. Formulation for controlling the infections caused by such phytopathogenic fungi as Pythium spp. and/or Fusarium spp., according to claim 4, comprising, besides Pseudomonas strains NCIMB 40403, NCIMB 40376, NCIMB 40375, NCIMB 40377, NCIMB 40402 and NCIMB 40379, inert supports selected from vermiculite, sepiolite, celite or kaolin.

6. Formulation for controlling the infections caused by such phytopathogenic fungi as Pythium spp. and/or Fusarium spp., according to claim 5, in which the inert support is coadjuvated by an adhesion promoter selected from among gum arabic, carboxymethylcellulose (CMS) or poly(vinyl alcohol) (PVA).

7. Formulation for controlling the infections caused by such phytopathogenic fungi as Pythium spp. and/or Fusarium spp., according to claim 6, in which the adhesion promoter is PVA N300.

8. Method for protecting growth and production of crops infested by Pythium spp. and/or Fusarium spp., which method consists of treating the seeds, the roots of said crops, or the soil on which said crops grow, with at least one strain of Pseudomonas, selected from among those strains which are identified as NCIMB 40403, NCIMB 40376, NCIMB 40375, NCIMB 40377, NCIMB 40402 and NCIMB 40379, in the form of cells, as such, or as suitable formulations.

9. Method for protecting growth and production of crops according to claim 8, in particular in the case of infections caused by Pythium ultimum and/or Fusarium oxysporum lycopersici.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | WO-A-9 105 475 (IMPERIAL CHEMICAL INDUSTRIES PLC) --- | | A01N63/00 C12N1/20 //(C12N1/20, C12R1:39) (C12N1/20, C12R1:40) |
| A | US-A-4 642 131 (H.A.J.HOITINK) --- | | |
| A | US-A-4 647 533 (D.M.WELLER ET. AL.) --- | | |
| A | US-A-4 714 614 (F.M.SCHER) --- | | |
| A | SCIENCE. vol. 216, 25 June 1982, LANCASTER, PA US pages 1376 - 1381 M.N.SCHROTH & J.G.HANCOCK 'Disease-Suppressive Soil and Root-Colonizing Bacteria' ----- | | |

TECHNICAL FIELDS
SEARCHED (Int. Cl.5 )

A01N
C12R

The present search report has been drawn up for all claims

| Place of search | Date of completion of the search | Examiner |
|---|---|---|
| THE HAGUE | 25 SEPTEMBER 1992 | DONOVAN T.M. |